# EUROPEAN PATENT SPECIFICATION

(11) **EP 1 656 389 B1**
(45) Date of publication and mention of the grant of the patent: **08.08.2007**
(21) Application number: 04742182.1
(22) Date of filing: 08.07.2004
(51) Int. Cl.: C07K 1/107

(54) **A METHOD FOR CLEAVING PROTEINS**
VERFAHREN ZUM SPALTEN VON PROTEINEN
METHODE DE CLIVAGE DE PROTEINES

(30) Priority: 09.07.2003 FI 20031050
(43) Date of publication of application: 17.05.2006
(73) Proprietor: VALTION TEKNILLINEN TUTKIMUSKESKUS, 02150 Espoo (FI)
(72) Inventor: LINDER, Markus, FIN-00200 Helsinki (FI); JÄNTTI, Jussi, FIN-00160 Helsinki (FI); SÖDERLUND, Hans, FIN-02940 Espoo (FI); HELAJA, Juho, FIN-00740 Helsinki (FI); KOSKINEN, Ari, FIN-03600 Karkkila (FI)
(74) Representative: Knuth-Lehtola, Sisko Hillevi
(86) International application number: PCT/FI2004/000439
(87) International publication number: WO 2005/005458

(56) References cited:
- WO-A1-98/29109
- HUMPHREYS D.P. ET AL: 'Improved efficiency of site-specific copper(II) ion-catalysed protein cleavage effected by mutagenesis of cleavage site' PROTEIN ENGINEERING vol. 13, no. 3, 2000, pages 201 - 206, XP002903828
- ALLEN G. ET AL: 'Specific cleavage of histidine-containing peptides by copper(II)' INT. J. PEPTIDE PROTEIN RES. vol. 48, 1996, pages 265 - 273, XP000623834
- EL-KHAWAGA O.Y. ET AL: '2-Methylaminopyridine-copper(II) Complex Catalyzes Protein Degradation' JOURNAL OF BIOCHEMISTRY, MOLECULAR BIOLOGY AND BIOPHYSICS vol. 6, no. 6, 2002, pages 433 - 436, XP002903829
- DATABASE MEDLINE (NLM) [Online] TACHON P.: 'Ferric and cupric ions requirement for DNA single-strand breakage by H2O2', XP002903830 Database accession no. 2509299 & FREE RADICAL RESEARCH COMMUNICATIONS vol. 7, no. 1, 1989, SWITZERLAND, pages 1 - 10
- KAMINSKAIA N.V. ET AL: 'New selectivity in peptide hydrolysis by metal complexes. Platinum(II) complexes promote cleavage of peptides next to the tryptophan residue' INORG. CHEM. vol. 40, 2001, pages 2368 - 2377, XP002903831
- BAL W. ET AL: 'Ni(II) specifically cleaves the C-terminal tail of the major variant of histone H2A and forms an oxidative damage-mediating complex with the cleaved-off octapeptide' CHEM. RES. TOXICOL. vol. 13, 2000, pages 616 - 624, XP002903832

## Description

This invention relates to a method for cleaving proteins at a predetermined, specific site. In particular, the invention concerns a method for cleaving recombinant proteins.

### Background

Biotechnical and especially pharmaceutical industry is developing proteins with desirable properties for bulk, diagnostic, clinical and research use. Often it is not possible or economically beneficial to isolate bioactive polypeptides from natural sources in large quantities with high purity. To circumvent this problem both prokaryotic and eukaryotic production systems have been developed for expression of the desired proteins in high quantities. The produced recombinant proteins are only rarely secreted out from the production host enabling purification of the protein from the often low protein containing growth medium. In most cases the recombinant proteins are produced inside the production host. In either case the desired target protein is often found as minor component in a complex mixture.

In order to facilitate the purification of recombinant proteins from the production host proteins, specific polypeptide tags, non-existing in the host cell proteins, are added to the target proteins by molecular biological methods. These tags bind with high affinity to a well defined immobilized ligand and many such systems are commercially available. These systems enable efficient purification of the recombinant protein from production host lysates or supernatants, optimally in a single affinity purification step. The alternative to using affinity tags is to purify proteins by chromatographical methods typically involving size exclusion, hydrophobic interactions and ion exchange. This alternative is characterized by high costs and tedious optimization.

The main drawback of affinity tag usage is that frequently, the tag has to be removed after purification of the target protein. This need for tag removal can be due to the undesirable effects the tag can have on the biological activity of the target protein or in the case of pharmaceutical/clinical applications where there is a need to remove any foreign protein sequences that might e.g. cause an immune response in patients.

The removal of affinity tags from purified proteins is usually carried out by protease cleavage. The most popular and specific proteases used are blood coagulation factors and their corresponding cleavage sites. Beneficially this system offers ways to very specifically define the cleavage site. However, it has become evident that use of proteases is often very inefficient or entirely non-functional. Furthermore, the enzymes needed are often expensive thus increasing the production costs. Currently cleavage is mainly carried out with proteases that are of high cost, and thus not feasible for large-scale use. This has presumably in part slowed down the utilization of recombinant proteins in different biotechnological applications.

Chemical methods offer the advantage of being potentially fast, cheap and quantitative. Only a few reactions have been described in the literature, of which probably the best known is the specific cleavage after a methionine by cyanogen bromide (Protein Sequence Analysis, 2nd Edition, L.R. Croft, John Wiley and Sons; B. Witkop, Advan. Prot. Chem. 16 (1961), 221). It has the drawbacks that it requires a very low pH, the reagents are highly toxic and the recognition site is a single methionine which does not offer sufficient specificity.

US 4,644,057 describes the cleavage of peptides and proteins at the methionyl bond using cyanogen chloride. This method results in cleavage at peptide sequences Met-X (X any amino acid). Proteins frequently contain internal Met residues resulting in undesirable fragmentation in cleavage with cyanogen chloride. Cyanogen chloride is also toxic.

EP 288272 B 1 (or US 4,745,178) describes a process for selectively cleaving a peptide or protein at one or more of its tryptophan residues. The process comprises treating the peptide or protein in trifluoroacetic acid with an organic sulfoxide, chloride ion and water. Proteins frequently contain trypthophan residues, and therefore this method is likely to result in undesirable protein fragmentation. In addition, the method is likely to result in protein denaturation due to the acidic conditions.

WO 9528415 suggests a method of cleaving a protein or peptide in an Asn-Gly bond thereof, wherein the protein or peptide is treated with a compound of the general formula (I): R ₁-(CH₂ₙ)-NH-(CH₂) ₘ-R₂, wherein R₁ is NH₂ or OH, R₂, is hydrogen, lower alkyl, NH₂, OH or halo, n is an integer from 1 to 3, and m is 0 or an integer from 1 to 3. Asn-Gly sequences are frequently found in proteins. Cleavage with the proposed agent is likely to generate non-specific protein fragmentation.

Kim et al. 1985 has studied the role of oxidative damage in enzyme degradation. Some enzymes are inactivated and degraded in the presence of dithiothreitol (DTT), oxygen and catalytic amounts of iron salts. The roles of DTT and iron can be replaced by ascorbate and copper. Since metal ions are common contaminants in many laboratory biochemicals, oxidative damage may cause the degradation of enzymes in a buffer containing DTT. Although Kim et al. studied the role of metal ions in degrading enzymes he did not suggest any use of metal ions for specific cleavage of proteins. Instead, the role of metal chelates in cleaving proteins and peptides has been studied.

Iron chelates can upon induction with ascorbate and H₂O₂ cleave a near-by peptide bond. Rana and Meares (1991) report of experiments where in the presence of ascorbate and H₂O₂, an iron chelate attached to Cys-212 of the enzyme human carbonic anhydrase I cleaved the protein between residues Leu-189 and Asp-190 to produce two distinct fragments. The authors of the publication concluded that the observed chelate-mediated proteolysis apparently does not depend on the chemical reactivity of the amino acid to be cleaved; besides the Leu/Asp hydrolysis of HCAI, the cleavage of bovine serum albumine occurred between Ala-150 and Pro-151 and between Ser-190 and Ser-191. Since the cleavage seems to be nonspecific, and the cleavage site cannot be predicted, the cleavage cannot be directed to a preselected site in a protein. The addition of Cys residues is often not practical since it may result in problems in the production and the stability of the protein caused by disulfide formation and other reactions of the sulfhydryl group.

US Patent Application No. 20020165365 describes a synthetic catalyst of the formula (A) which can selectively recognize and cleave a specific protein among a protein mixture, and a method for selective cleavage of a target protein using the formula (A): (R)(Z)ₙ, in which n denotes an integer of 1 or more, R represents a material capable of selectively recognizing and binding a target protein and Z represents a metal ion-ligand complex. This is not a significant improvement from the previous (such as Rana and Meares (1991)) since it is not a practical and general solution for directing the cleavage to a predetermined site in a protein.

Some publications disclose the cleavage of proteins in the presence of metal ions and an oxidative agent. For example, Chiou, S.-H. 1983 (J.Biochem. 94:1259-1267) discloses the cleavage of DNA and proteins in the presence of ascorbate and copper or iron ions and WO 9829109 discloses the cleavage of autoantigens with immunocryptic sites in the presence of iron and copper and reactive oxygen species. However, the cleavage in these experiments did not occur at a specific, designed site of the protein. Some publications disclose specific cleavage of proteins in the presence of metal ions, but without the use of an oxidative agent. For example, WO 0032795 and Humpreys et al. 2000 (Protein Engineering 13(3):201-206) disclose the cleavage of ^{N}DKTH^{C} peptide between Lys and Thr residues by Cu⁺⁺ ions. Conservative mutations to three of the residues in the introduced cleavage site resulted in improved cleavage by Cu⁺⁺ ions and also by Ni⁺⁺ ions. According to the authors, by the inclusion of H₂O₂ with and without ascorbic acid, ascorbic acid alone, and some other agents, no increase in cleavage was found. The publication by Allen and Campbell, 1996 ( Int. J. Peptide Protein Res. 48:265-273) discloses that Copper (II) cleaves with moderate specificity peptides containing Ser-His or Thr-His sequences. According to Allen and Campbell, the cleavage is clearly different from the oxidative degradation of proteins catalysed by copper ions.

In spite of the above mentioned developments, there is still a need for a general method for cleaving proteins (polypeptides or peptides) at a predetermined, specific site in a protein. In particular, there is a need for a practical cleavage method where a site can be engineered and inserted into a protein or polypeptide, and a reagent which will cleave the protein or polypeptide at the specific site. It would be important that the cleaved protein or peptide retains its activity as well as possible. Furthermore, it would be of advantage if the reaction could be carried out without toxic or harmful chemicals.

### Summary

It is an aim of the present invention to eliminate the problems associated with the prior art. This invention provides a method for cleaving a protein or peptide at a specific site. The specific cleavage site can be constructed into a protein or peptide at a desired site.

The present invention is based on the surprising finding that certain amino acids are cleavable in the presence of free metal ions in solution. According to the invention, a predetermined, specific amino acid sequence comprising these selected amino acids is constructed, or inserted, into a protein or peptide at a specific site. The protein or peptide with the constructed or inserted site is then allowed to react with free metal ions in buffer. The desired cleaved protein part may then be recovered from the solution, if desired.

More specifically, the method according to the invention is characterized by what is stated in the characterizing part of claim 1.

According to this invention, a predetermined, specific cleavage site comprising amino acids that are cleavable in the presence of free metal ions is constructed into a protein or peptide. The metal ions preferably belong to the group of transition metals. More preferably the metal ions are ions of metals selected from the group comprising Cu, Co, Ni , Fe, Mn, Cd, Pd, Rh, Ru, Pt, Cr and Zn, still more preferably from the group comprising Cu, Co, Mn, Cr, Ni , Fe and Zn. The most preferred ions are those of the metals Cu and Co.

The protein or peptide to be cleaved is allowed to react with a metal salt in a buffer in near neutral pH. The reaction is carried out preferably at pH 3 -9.

A great advantage of the invention is that the cleavage occurs specifically at the constructed or inserted site. In addition, there is no need to prepare synthetic metal chelates of the metal ions. The activity of cleaved protein is very well retained. Furthermore, other advantages of the invention are that the reaction can be carried out on broad pH range and that the reagents needed are not toxic.

According to the prior art, for example Humphreys et al. 2000, redox active compounds do not have any advantageous effect to the cleavage process. In this invention it has surprisingly been found that specific cleavage occurs preferably in the presence of oxidizing or reducing (redox active) compounds. Humphreys et al. use pHs well above pH 7, temperatures over 55 °C and reaction times over 10 hours. Advantages of the present invention are broader pH range (pHs from 3 to 9), lower temperatures (+4 °C and higher), shorter reaction times (15 minutes and longer).

By the method of this invention any protein or peptide can be cleaved. The protein may be generally a recombinant protein or a fusion protein. Cleavage may be desirable to activate or inactivate the protein, or to release a segment of the protein. The segment may, for example, be used for identification of the protein or other molecule that is linked or bound to it.

Furthermore, the method of this invention is specific and quick. The invention is useful for the pharmaceutical and other biotechnological industry, which is increasingly making use of different affinity-tags to aid the purification of the desired recombinant protein products. Use of recombinant fusion proteins cuts costs of downstream processing, increases production yields and shortens the time required for process development. Thus, their exploitation is expected to increase in the future.

Next the invention will be examined more closely with the aid of a detailed description and a number of working examples.

### Brief description of the drawings

Figure 1. A map of the *E*. *coli* expression plasmid pLink1 for MBP-ABP.
Figure 2. A map of the *E*. *coli* expression plasmid pLink2 for MBP-Gly-Ser-Pro-Thr-Gly-Ala-Ser-Thr-His-His-His-His-His-His-Gly-Ser-Pro-Thr-Gly-Ala-Ser-Thr-ABP (SEQ ID NO: 1).
Figure 3. A map of the *E. coli* expression plasmid pLink3 for MBP-Gly-Ser-Pro-Thr-Gly-Ala-Ser-Thr-Gly-Gly-Gly-Gly-Gly-Gly-Gly-Ser-Pro-Thr-Gly-Ala-Ser-Thr-ABP (SEQ ID NO: 2).
Figure 4. A map of the *E*. *coli* expression plasmid pLink6 for MBP-Gly-Ser-Pro-Thr-Gly-Ala-Ser-Thr-His-His-His-His-His-His-Gly-Ser-Pro-Thr-Gly-Ala-Ser-Thr-Avidin (SEQ ID NO: 3).
Figure 5. A map of the *E*. *coli* expression plasmid pLink7 for MBP-Gly-Ser-Pro-Thr-Gly-Ala-Ser-Thr-Gly-Ser-Thr-Gly-Pro-Ser-Gly-Ser-Pro-Thr-Gly-Ala-Ser-Thr-Avidin (SEQ ID NO: 4).
Figure 6. A map of the *E*. *coli* expression plasmid pLink8 for MBP-Gly-Ser-Pro-Thr-Gly-Ala-Ser-Thr-His-His-His-His-Gly-Ser-Pro-Thr-Gly-Ala-Ser-Thr-Avidin (SEQ ID NO: 5).
Figure 7. A map of the *E*. *coli* expression plasmid plink10 for MBP-Gly-Ser-Pro-Thr-Gly-Ala-Ser-Thr-His-His-Gly-Ser-Pro-Thr-Gly-Ala-Ser-Thr-Avidin (SEQ ID NO: 6).
Figure 8. A map of the *E*. *coli* expression plasmid pLink12 for MBP-Gly-Ser-Pro-Thr-Gly-Ala-Ser-Thr-His-His-His-His-His-His-His-His-Gly-Ser-Pro-Thr-Gly-Ala-Ser-Thr-Avidin (SEQ ID NO: 7).
Figure 9. A map of the *E*. *coli* expression plasmid pLink13 for MBP-Gly-Ser-Pro-Thr-Gly-Ala-Ser-Thr-His-Ser-His-Ala-His-Gly-His-Ala-His-Ser-His-Gly-Ser-Pro-Thr-Gly-Ala-Ser-Thr-Avidin (SEQ ID NO: 8).
Figure 10. Cleavage with free Cu²⁺ ions of equimolar concentration, MBP-His6-AVI (lanes 2 and 3) and control protein with no His-residues in the linker (lanes 4 and 5).
Figure 11. Cleavage with free Cu²⁺ ions, the effect of copper ion concentration: no copper (lanes 1 and 5), ~equimolar (lanes 2 and 7), 5 equivalents (lanes 3 and 8), 10 equivalents (lanes 4 and 9), and 17 equivalents (lanes 5 and 10). In lanes 1-5 the protein has a cleavage site and in 6-10 there is no cleavage site.
Figure 12. Cleavage with free Cu²⁺ (a), Ni²⁺ (b), Fe³⁺ (c), Zn²⁺ (d), and Co²⁺ (e) ions. In lanes 1-5 the protein has a cleavage site and in 6-10 there is no cleavage site.
Figure 13. Effect of ascorbate (lanes 3-9) or hydrogen peroxide (lanes 12-18) on cleavage of MBP-His6-AVI (lanes 1 and 10).
Figure 14. Effect of combinations of ascorbate and hydrogen peroxide on cleavage of MBP-His6-AVI.
Figure 15. Effect of buffer on cleavage.
Figure 16. The effect of pH on cleavage.
Figure 17. Cleavage with free Cu²⁺ ions, the effect of amount of histidines in linker: no His (lanes 2 and 3), 2 His (lanes 4 and 5), 4 His (lanes 6 and 7) (SEQ ID NO:28), and 6 His (lanes 8 and 9) (SEQ ID NO:29).
Figure 18. The effect of amount of histidines in linker: 6xHis (lanes 1 and 2), 8xHis (lanes 3 and 4) (SEQ ID NO:30), 6 His within the segment His-Ser-His-Ala-His-Gly-His-Ala-His-Ser-His-Gly (SEQ ID NO: 9) (lanes 5 and 6), molecular weight marker (lane 9).

### Detailed description of the invention

By "a protein" is meant here any polypeptide or peptide that is intended to be cleaved at a specific site. In a typical case the protein is a recombinant protein that needs to be separated from a purification tag or a fusion protein which needs to be cleaved at the fusion site to separate the fusion part from the protein part of interest. By a protein is meant a functional entity. "Peptides" are substances composed of two or more amino acids and designated as di- tri- oligo- or polypeptides according to the number of amino acids linked by peptide bond.

By an "amino acid sequence" is in this invention meant a sequence comprising amino acids that are cleavable in the presence of free metal ions. The amino acids are preferably selected from the group comprising histidine (His), lysine (Lys), tryptophan (Trp), arginine (Arg), tyrosine (Tyr), phenylalanine (Phe), and cysteine (Cys). In the amino acid sequence there are preferably at least two of said amino acids and the distance of said amino acids is preferably less than three amino acids. The length of the amino acid sequence is preferably less than 100 amino acids, more preferably less than 50 amino acids, but the amino acid sequence may also be part of a longer amino acid sequence.

By "a specific cleavage site" is meant that the polypeptide or peptide is cleaved at a specific predetermined position. Such cleavage site can be constructed to a predetermined position of the protein or peptide according to the invention. If the protein is a fusion protein, the cleavage site is likely to be constructed at the fusion site. In other applications the engineered cleavage site may be in any position of the protein or peptide where cleavage will affect structure or function of the protein or peptide or where some part of the protein or peptide needs to be removed.

By "metal ions" is here meant free metal ions of a metal salt. Preferred metal ions in the method of this invention are transition metal ions, preferably selected from the group comprising Cu, Co, Ni, Fe, Mn, Cd, Pd, Rh, Ru, Pt, Cr and Zn, more preferably from the group comprising Cu, Co, Mn, Cr, Ni , Fe and Zn. More preferably they are ions of metals selected from the group comprising Cu and Co or they are metal ions the function of which is similar to the function of Cu²⁺ and Co²⁺. The choice of metal ion added to the reaction mixture seems to have quite a large effect on the reaction as shown in example 5. Cu²⁺ was the most effective followed by Co²⁺, but Zn²⁺, Fe³⁺, and Ni²⁺ were not as active as Cu²⁺ and Co²⁺.

In the reaction of this invention the molar ratio of metal ions to protein is preferably between 10:1 to 1:10. More preferably the molar ratio is between 5:1 to 1:5, most preferably between 2:1 to 1:2.

The anion in the metal salt can be sulphate, chloride, nitrate or any other forming a soluble salt. The selection of the anion seems not to be critical for the reaction.

Amino acids cleavable in the presence of metal ions are preferably amino acids that show activity for binding of metals. Preferably the amino acids are selected from the group comprising histidine, lysine, tryptophan, arginine, tyrosine, phenylalanine, and cysteine.

More preferably they are selected from the group comprising histidine, lysine, tryptophan and cysteine. The amino acid sequences constructed to the predetermined cleavage site can comprise units comprising the preferred amino acids in different combinations or repeats of the units. Alternatively the amino acid sequences may comprise the preferred amino acids with other amino acids inserted in between. The other amino acids may be any amino acids or they may be amino acids neutral in their capability of binding metals.

The preferred amino acid sequence comprises repeats of 2, 4, 6 or 8 His residues. The histidine may be replaced or combined with the other preferred amino acids. According to a preferred embodiment of the invention,the amino acid sequence comprises His residues with 1 to 3, 1 to 2 or 1 amino acid in between.

The length of the amino acid sequence is preferably less than 100 amino acids, more preferably 2 to 50 amino acids. Longer sequences can be used, but they are of no significant advantage. Most useful are sequences having 2 to 20, preferably 2 to 10 amino acids. Good results are obtained by sequences having 4 to 6 or 4 to 8 amino acids.

In the experimental part of the invention is shown that His-residues in a peptide segment promote cleavage of the segment in the presence of for example Cu ions and other reagents such as ascorbate and hydrogen peroxide. As shown in the examples (example 9) two adjacent His residues already causes cleavage. Increasing this number to 4, 6 or 8 increases the reactivity of the segment (Example 9). Surprisingly, a segment with six His residues that have some other residue between each of the His residues is also very reactive, and may be even more reactive than six consecutive His residues (example 10). In the example the sequence His-Ser-His-Ala-His-Gly-His-Ala-His-Ser-His-Gly (SEQ ID NO:9) was used. This shows that there is potentially a large number of possible variations in the sequence that can be used in the invention. Amino acid residues, such as Cys, Trp and Lys that can be functionally similar in this respect to His, are shown to be especially suitable. However, cysteines might cause problems due to disulfide formation. This can be avoided by adding other free sulfhydryl containing compounds.

The cleavage occurs at a wide range of pH. No significant difference is seen between pH 3-9. Increasing the temperature has the effect of speeding up the reaction, but ambient temperature can conveniently be used. The reaction is also not very sensitive to the choice of buffer. Examples of useful buffers are ammoniun acetate, Tris, Hepes, and phosphate buffered saline. The use of different buffers and the effect of pHs are exemplified in examples 7 and 8.

By oxidizing and/or reducing compounds or agents is here meant redox active compounds or agents. The reaction may be carried out in the presence of one or more redox active compounds or agents, alone or in combination. Additives, such as ascorbate, hydrogen peroxide and dithiothreitol is of advantage, since they increase the rate of peptide segment cleavage. The presence of, for example, ascorbate and hydrogen peroxide, is shown to increase the rate of peptide segment cleavage in example 6. It is possible that other reducing or oxidizing compounds (redox active or antioxidant compounds), such as glutathione, carotene, or other sulfhydryl containing compounds, may function similarly.

The amino acid sequence is constructed to the predetermined cleavage site by molecular biology methods at the nucleic acid level. Molecular biology methods which can be used for constructing the cleavage sites are well-known to a person skilled in the art and are described in Sambrook et al. 1989.

The following examples are for illustration of the present invention and should not be construed as limiting the present invention in any manner.

### Example 1

### Construction of vectors for expression of Maltose Binding Protein (MBP)-linker-Albumin Binding Protein (ABP)

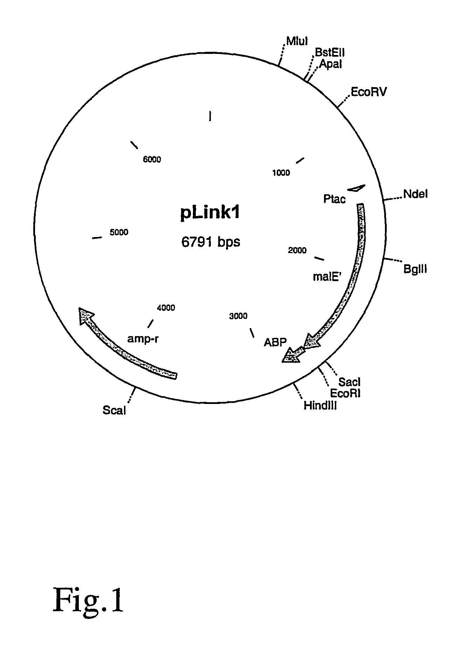

For construction of a vector expressing *Escherichia coli* Maltose Binding Protein (MBP) as a fusion protein with a peptide linker and a fragment of the Albumin Binding Protein (ABP) (MBP-linker-ABP) in *Escherichia coli,* the DNA fragment encoding ABP was pcr amplified from vector pKNl (Nord et al., 1995) with the 5' oligo GCATTGGATTCGAATTCTTAGCTGAAGCTAAAGTCTTAGC (EcoRI sequence underlined, SEQ ID NO: 10) and 3' oligo GCATTAAGCTTCTATTCGCTTTTTGCCGGAGTAG (HindIII sequence underlined, (SEQ ID NO:11). These oligos amplify a fragment encoding amino acids 496-541 of the *Staphylococcus carnosus* cell surface protein (GeneBank U15515) albumin binding domain. The pcr fragment was ligated to the pCR2.1-TOPO vector (In Vitrogen). This vector was cut with EcoRI and HindIII and the DNA fragment ancoding ABP was ligated into EcoRI and HindIII cut pMal-c2X (New England Biolabs) vector to yield pLink1 (Figure 1).

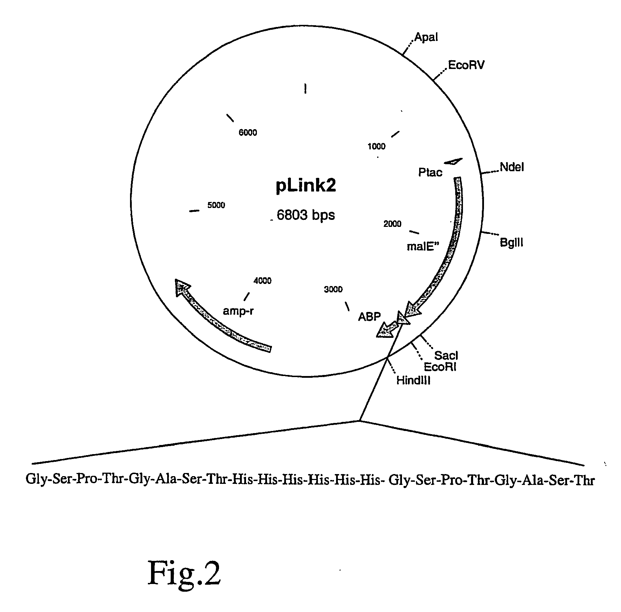

To add the sequence encoding for the linker Gly Ser Pro Thr Gly Ala Ser Thr His His His His His His Gly Ser Pro Thr Gly Ala Ser Thr (SEQ ID NO:1) in between MBP and ABP in pLink1, the 5' oligo
CGGGTAGCCCAACCGGCGCGAGCACCCATCACCATCACCATCACGGTAGCCCA ACCGGCGCGAGCACCG (SEQ ID NO:12) and 3' oligo AATTCGGTGCTCGCGCCGGTTGGGCTACCGTGATGGTGATGGTGATGGGTGCT CGCGCCGGTTGGGCTACCCGAGCT (SEQ ID NO:13) were kinased in vitro by T4 polynucleotide kinase, annealed and ligated into SacI and EcoRI cut pLink1. This generated pLink2 (Figure 2).

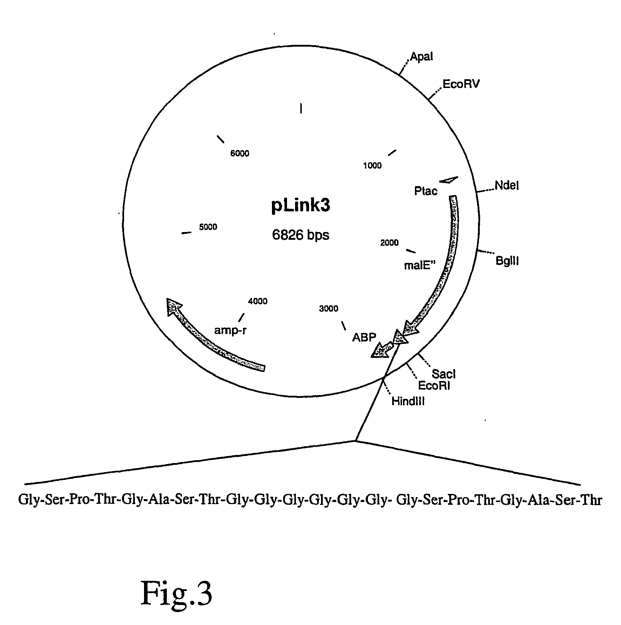

To generate a vector expressing MBP-Gly Ser Pro Thr Gly Ala Ser Thr Gly Gly Gly Gly Gly Gly Gly Ser Pro Thr Gly Ala Ser Thr-ABP (SEQ ID NO:2), the 5' oligo CG GGT AGC CCA ACC GGC GCG AGC ACC GGC GGT GGT GGT GGC GGC GGT AGC CCA ACC GGC GCG AGC ACC G (SEQ ID NO: 14) and 3' oligo AATTCGGTGCTCGCGCCGGTTGGGCTACCGCCGCCACCACCAGGGCCGGTGCT CGCGCCGGTTGGGCTACCCGAGCT (SEQ ID NO:15) were kinased in vitro by T4 polynucleotide kinase, annealed and ligated into pLink1 . This generated pLink3 (Figure 3).

### Example 2

### Construction of vectors for expression of Maltose Binding Protein (MBP)-linker-Avidin

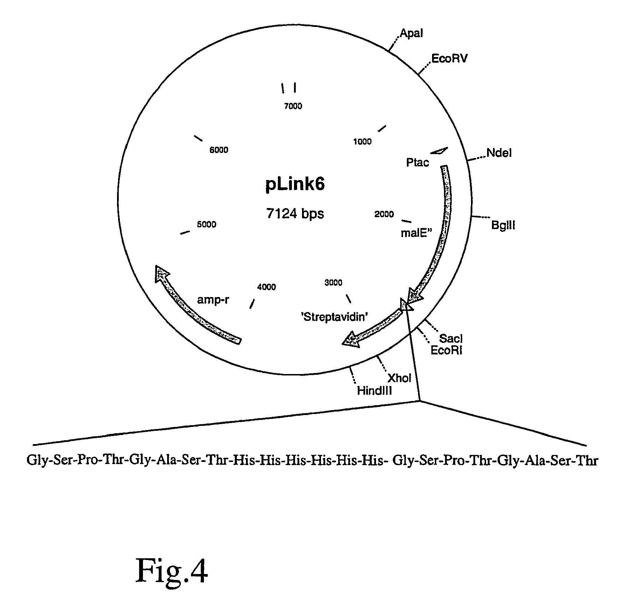

For construction of a vector for expression of MBP-Gly Ser Pro Thr Gly Ala Ser Thr His His His His His His Gly Ser Pro Thr Gly Ala Ser Thr-Avidin (SEQ ID NO:3) in Escheria coli, a DNA fragment of the Streptomyces avidinii gene for amino acids 25-183 of streptavidin (GeneBank X03591) was amplified by pcr by using the vector pK501-1 (C. Oker-Blom et al.,1996. FEBS letters, 389, 238-243) as a template. The 5' oligo was GCATTGAATTCGACCCCTCCAAGGACTCGAAGG (SEQ ID NO: 16) and the 3' oligo was GCATTAAGCTTCTACTGCTGAACGGCGTCGAGC (SEQ ID NO:17). The pcr fragment was TA-ligated to the pCR2.1-TOPO vector (InVitrogen) and subsequently after cutting with EcoRI and HindIII enzymes ligated into EcoRI and HindIII cut pLink2 vector to yield pLink6 (Figure 4).

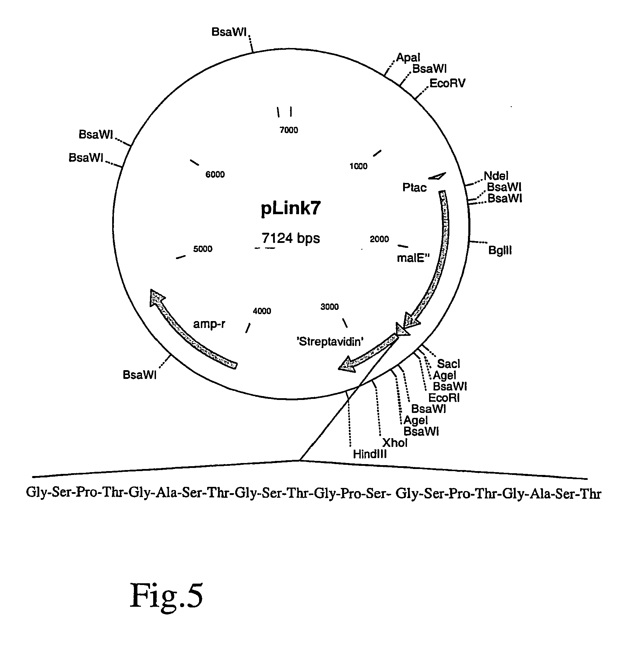

To generate a vector expressing MBP-Gly Ser Pro Thr Gly Ala Ser Thr Gly Ser Thr Gly Pro Ser Gly Ser Pro Thr Gly Ala Ser Thr-Avidin (SEQ ID NO:4) the 5' oligo CGGGTAGCCCAACCGGCGCGAGCACCGGCAGCACCGGTCCAAGCGGTAGCCC AACCGGCGCGAGCACCG (SEQ ID N0:18) and the 3' oligo AATTCGGTGCTCGCGCCGGTTGGGCTACCGCTTGGACCGGTGCTGCCGGTGCTC GCGCCGGTTGGGCTACCCGAGCT (SEQ ID NO: 19) were kinased in vitro by T4 polynucleotide kinase, annealed and ligated into SacI and EcoRI cut pLink6. This generated pLink7 (Figure 5).

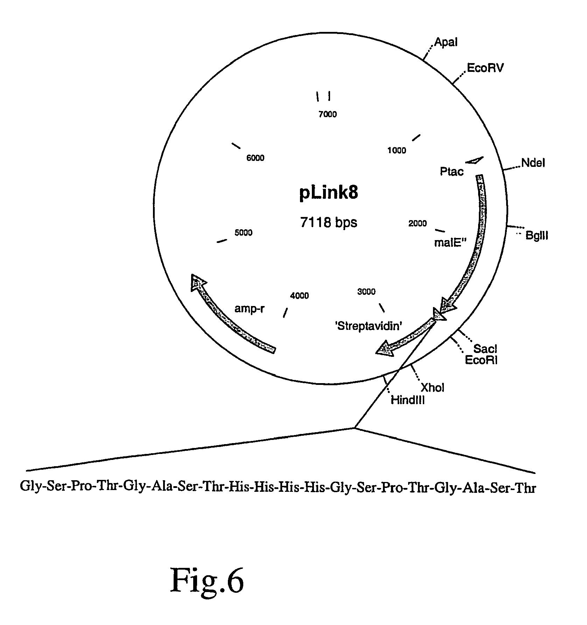

To generate a vector expressing MBP-Gly Ser Pro Thr Gly Ala Ser Thr His His His His Gly Ser Pro Thr Gly Ala Ser Thr-Avidin (SEQ ID NO:5) the 5' oligo CGGGTAGCCCAACCGGCGCGAGCACCCATCACCATCACGGTAGCCCAACCGGC GCGAGCACCG (SEQ ID NO:20) and the 3' oligo AATTCGGTGCTCGCGCCGGTTGGGCTACCGTGATGGTGATGGGTGCTCGCGCC GGTTGGGCTACCCG (SEQ ID NO:21) were kinased in vitro by T4 polynucleotide kinase, annealed and ligated into SacI and EcoRI cut pLink6. This generated pLink8 . (Figure 6).

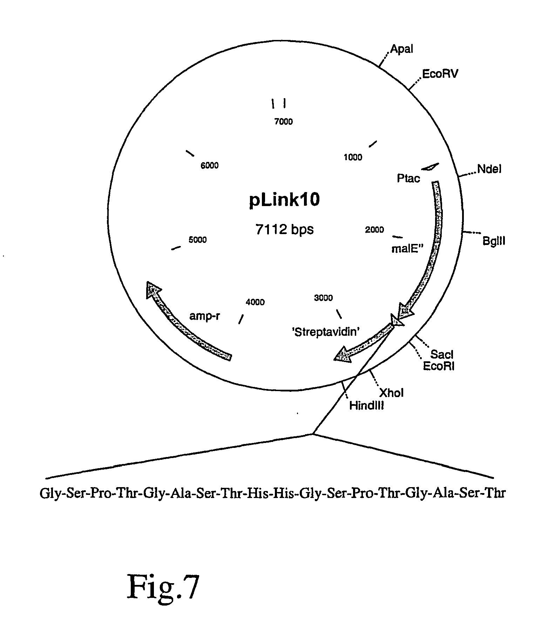

To generate a vector expressing MBP-Gly Ser Pro Thr Gly Ala Ser Thr His His Gly Ser Pro Thr Gly Ala Ser Thr-Avidin (SEQ ID NO:6) the 5' oligo CGGGTAGCCCAACCGGCGCGAGCACCCATCACGGTAGCCCAACCGGCGCGAG CACC (SEQ ID NO:22) and the 3' oligo AATTCGGTGCTCGCGCCGGTTGGGCTACCGTGATGGGTGCTCGCGCCGGTTGG GCTACCCGAGCT (SEQ ID NO:23) were kinased in vitro by T4 polynucleotide kinase, annealed and ligated into SacI and EcoRI cut pLink6. This generated plink10 (Figure 7).

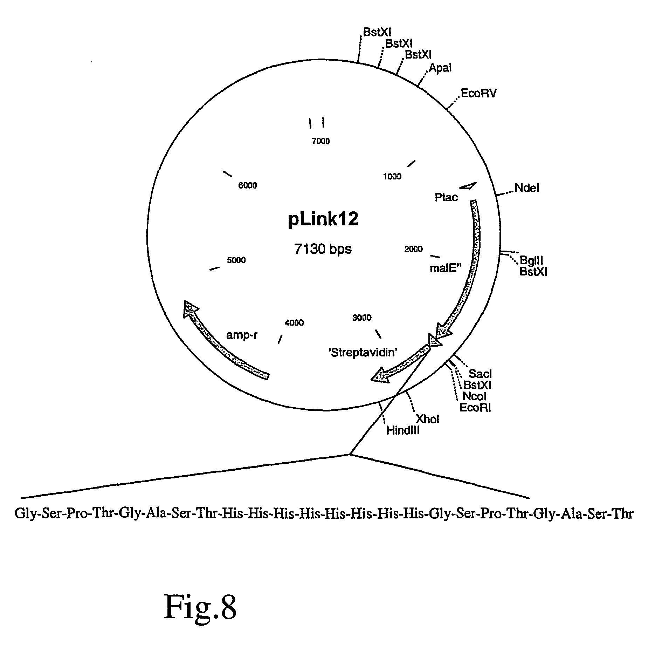

To generate a vector expressing MBP-Gly Ser Pro Thr Gly Ala Ser Thr His His His His His His His His Gly Ser Pro Thr Gly Ala Ser Thr-Avidin (SEQ ID NO:7) the 5' oligo CGGGTAGCCCAACCGGCGCGAGCACCCACCATCACCATCACCATCACCATGGT AGCCCAACCGGCGCGAGCACCG (SEQ ID NO:24) and the 3' oligo AATTCGGTGCTCGCGCCGGTTGGGCTACCATGGTGATGGTGATGGTGATGGTG GGTGCTCGCGCCGGTTGGGCTACCCGAGCT (SEQ ID NO:25) were kinased in vitro by T4 polynucleotide kinase , annealed and ligated into SacI and EcoRI cut pLink6. This generated pLink 12 (Figure 8).

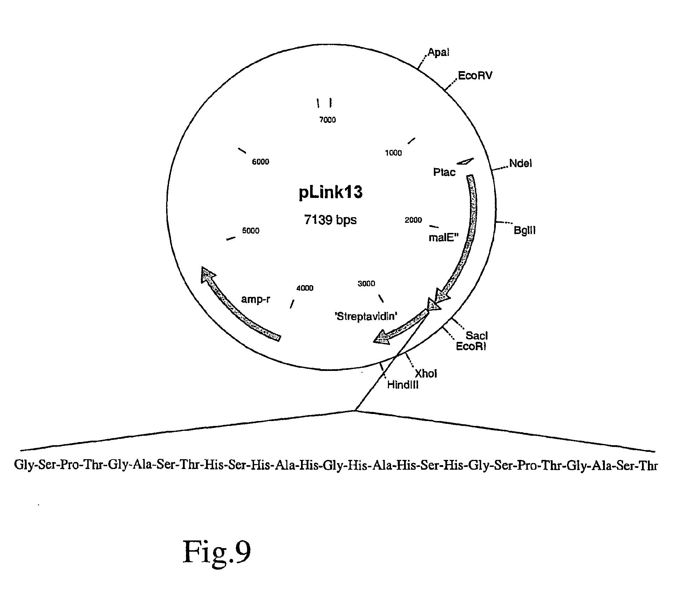

To generate a vector expressing MBP-Gly Ser Pro Thr Gly Ala Ser Thr His Ser His Ala His Gly His Ala His Ser His Gly Ser Pro Thr Gly Ala Ser Thr-Avidin (SEQ ID NO:8) the 5' oligo CGGGTAGCCCAACCGGCGCGAGCACCCATAGCCACGCGCATGGCCACGCGCA TAGCCACGGTAGCCCAACCGGCGCGAGCACCG (SEQ ID NO:26) and the 3' oligo AATTCGGTGCTCGCGCCGGTTGGGCTACCGTGGCTATGCGCGTGGCCATGCGC GTGGCTATGGGTGCTCGCGCCGGTTGGGCTACCCGAGCT (SEQ ID NO:27) were kinased in vitro by T4 polynucleotide kinase, annealed and ligated into Sacl and EcoRI cut pLink6. This generated pLink13 (Figure 9).

### Example 3

### Cleavage of a linker, containing six consecutive histidines vs. control

The experiments were done with MBP-His6-AVI (produced by using pLink6), and MBP-His0-AVI (no cleavage site, produced by using pLink7) in 8.5 mM NH₄HCO₃/HAc buffer, pH 8.6. Copper ion (Cu²⁺) concentration was equimolar to the protein concentration. The final concentrations in the reaction mixture (35 µl) are shown in Table 1. Incubation time was 1h in RT.

**Table 1. Final concentrations in the reaction mixture (35 µl).**

| | 1 | 2 | 3 | 4 | 5 |
|---|---|---|---|---|---|
| LMW standard | x | | | | |
| 3.3 µM MBP-his6-AVI | | x | x | | |
| 3.3 µM MBP-his0-AVI | | | | x | X |
| 3.3 µM CuCl₂ | | | x | | X |
| 4.6 mM Ascorbate | | | x | | X |
| 1.1 mM H₂O₂ | | | x | | X |

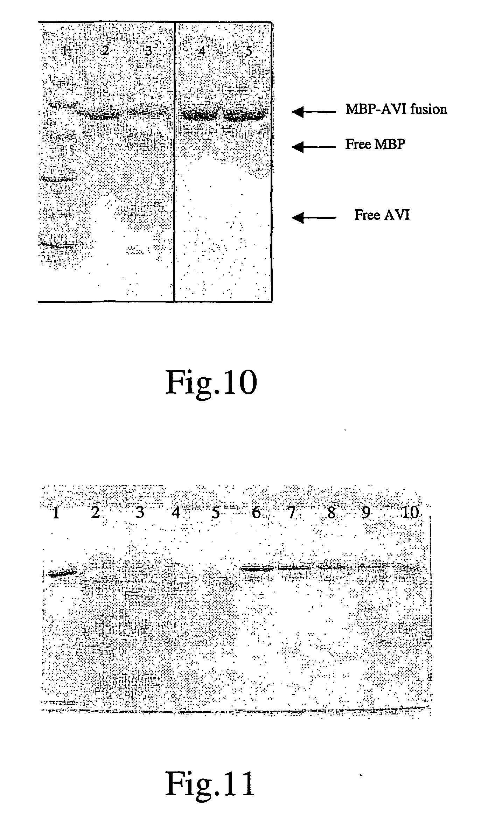

Result presented in figure 10. The protein with histidines in the linker is cleaved, whereas the control protein remains uncleaved.

### Example 4

### Effect of copper concentration

The experiment was done with MBP-His6-ABP (produced by using pLink2) and MBP-Gly-ABP (produced by using pLink3) in 8.5 mM NH₄HCO₃/HAc buffer, pH 8 varying Cu²⁺ion concentration starting with ~equimolar to protein concentration. The final concentrations in the reaction mixture (20 µl) are shown in Table 2. Incubation time was 15 min in +30 °C in all experiments.

**Table 2. Final concentrations in the reaction mixtures (20 µl).**

| | 1 | 2 | 3 | 4 | 5 | 6 | 7 | 8 | 9 | 10 |
|---|---|---|---|---|---|---|---|---|---|---|
| 0.6 µM MBP-His6-ABP | x | x | x | x | x | | | | | |
| 0.6 µM MBP-Gly-ABP | | | | | | x | x | x | x | X |
| 1 µM CuCl₂ | | x | | | | | x | | | |
| 3 µM CuCl₂ | | | x | | | | | x | | |
| 6 µM CuCl₂ | | | | x | | | | | x | |
| 10 µM CuCl₂ | | | | | x | | | | | X |
| 10 mM Ascorbate | | x | x | x | x | | x | x | x | X |
| 5 mM H₂O₂ | | x | x | x | x | | x | x | x | X |

Result in figure 11. Equimolar concentration of copper cleaves the fusion protein. Adding copper enhances the cleavage. Protein with His6 cleavage site is cleaved whereas protein without cleavage site (Gly) is not cleaved.

### Example 5

### Effect of different ions on cleavage efficiency

The experiments were done with MBP-His6-ABP (produced by using pLink2) and MBP-Gly6-ABP (produced by using pLink3) in 8.5 mM NH₄HCO₃/HAc buffer, pH 8. Metal ions under study were Cu²⁺, Ni²⁺, Fe³⁺, Zn²⁺, and Co²⁺. The final concentrations in the reaction mixture (20 µl) are shown in Table 3. Incubation time was 15 min in +30 °C in all experiments.

**Table 3. Final concentrations in the reaction mixtures (20 µl). Me = metal ion (Cu²⁺, Ni²⁺, Fe³⁺, Zn²⁺, or Co²⁺), the same conditions were used in all experiments.**

| | 1 | 2 | 3 | 4 | 5 | 6 | 7 | 8 | 9 | 10 |
|---|---|---|---|---|---|---|---|---|---|---|
| 0.6 µM MBP-His6-ABP x | x | x | x | x | x | | | | | |
| 0.6 µM MBP-Gly-ABP | | | | | | x | x | x | x | x |
| 1 µM Me | | x | | | | | x | | | |
| 3 µM Me | | | x | | | | | x | | |
| 6 µM Me | | | | x | | | | | x | |
| 10 µM Me | | | | | x | | | | | X |
| 10 mM Ascorbate | | x | x | x | x | | x | x | x | X |
| 5 mM H₂O₂ | | x | x | x | x | | x | x | x | X |

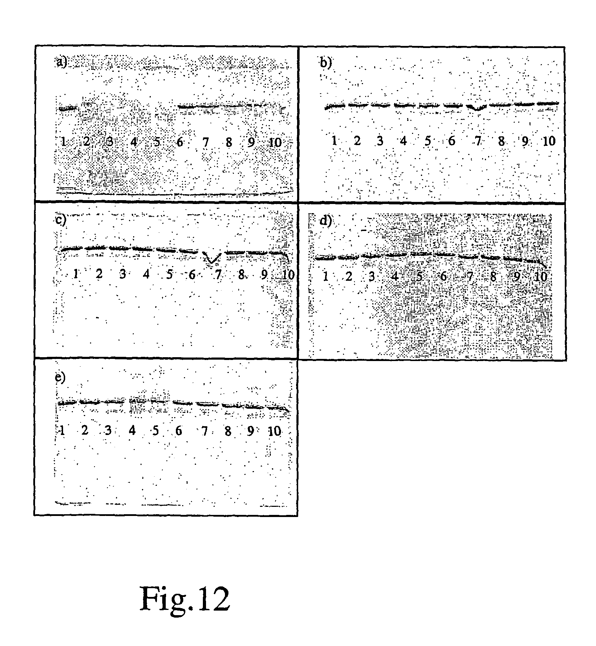

Results presented in figure 12. The cleavage of fusion protein containing six histidines in the linker sequence occurs most efficient with free copper ions while the reference protein remained untouched. Some cleavage can be observed with free cobalt ions.

### Example 6

### Effect of ascorbate and hydrogen peroxide concentration on cleavage

The effect of ascorbate and hydrogen peroxide concentration on cleavage was first studied separately and then together. The experiments were carried out with equimolar MBP-his6-AVI (produced by using pLink6) and copper ion concentration in 8.5 mM NH₄HCO₃/HAc buffer, pH 8.6. The final concentrations in the reaction mixture (35 µl) are shown in Table 4 (ascorbate and hydrogen peroxide separately) and in Table 5 (ascorbate and hydrogen peroxide together). Incubation time was ½ h in RT.

**Table 4. Final concentrations in the reaction mixture (20 µl) (Figure 4). Asc = ascorbate**

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| 1.9 µM MBP-AVI | x | x | x | x | x | x | x | x | x | x | x | x | x | x | x | X | x | x |
| 1.9 µm Cu | | x | x | x | x | x | x | x | x | | x | x | x | x | x | X | x | x |
| 0.2 mM Asc | | | x | | | | | | | | | | | | | | | |
| 2 mM Asc | | | | x | | | | | | | | | | | | | | |
| 4 mM Asc | | | | | x | | | | | | | | | | | | | |
| 8 mM Asc | | | | | | x | | | | | | | | | | | | |
| 12 mM Asc | | | | | | | x | | | | | | | | | | | |
| 16 mM Asc | | | | | | | | x | | | | | | | | | | |
| 20 mM Asc | | | | | | | | | x | | | | | | | | | |
| 0.05 mM H₂O₂ | | | | | | | | | | | | x | | | | | | |
| 0.2 mM H₂O₂ | | | | | | | | | | | | | x | | | | | |
| 1 mM H₂O₂ | | | | | | | | | | | | | | x | | | | |
| 2 mM H₂O₂ | | | | | | | | | | | | | | | x | | | |
| 3 mM H₂O₂ | | | | | | | | | | | | | | | | X | | |
| 4 mM H₂O₂ | | | | | | | | | | | | | | | | | x | |
| 5 mM H₂O₂ | | | | | | | | | | | | | | | | | | x |

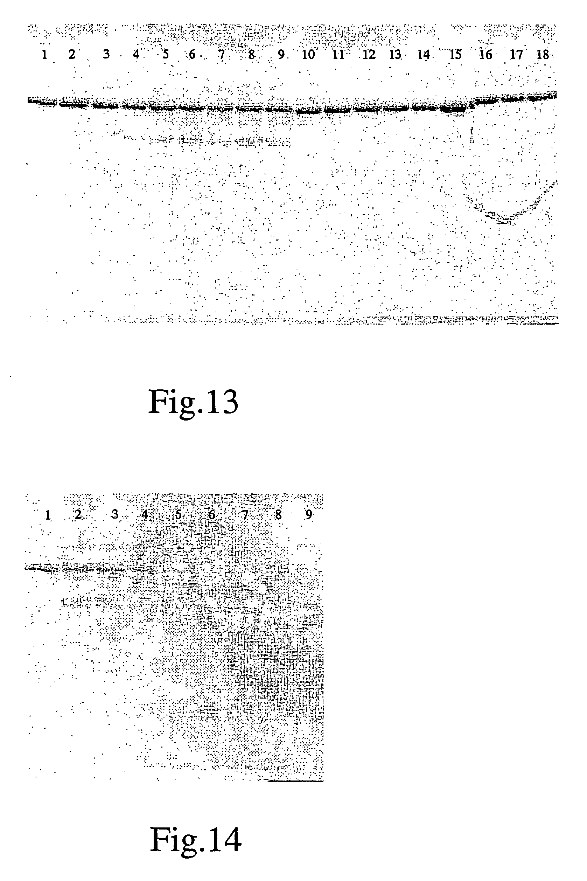

Results corresponding to table 4 are presented in figure 13.

**Table 5. Final concentrations in the reaction mixture (20 µl) (Figure 14).**

| | 1 | 2 | 3 | 4 | 5 | 6 | 7 | 8 | 9 |
|---|---|---|---|---|---|---|---|---|---|
| 1.9 µM MBP-AVI | x | x | x | x | x | x | x | x | x |
| 1.9 µM Cu | | x | x | x | x | x | x | x | x |
| 8 mM ascorbate | | x | x | x | x | x | x | x | x |
| 0.05 mM H₂O₂ | | | x | | | | | | |
| 0.2 mM H₂O₂ | | | | x | | | | | |
| 1 mM H₂O₂ | | | | | x | | | | |
| 2 mM H₂O₂ | | | | | | x | | | |
| 3 mM H₂O₂ | | | | | | | x | | |
| 4 mM H₂O₂ | | | | | | | | x | |
| 5 mM H₂O₂ | | | | | | | | | x |

Results corresponding to table 5 are presented in figure 14. Ascorbate is more efficient reagent than hydrogen peroxide on cleavage, but together they are most efficient. On the other hand, higher concentrations cause degradation of cleaved protein bands also.

### Example 7

### Effect of buffer at neutral pH

The effect of buffer at ~neutral pH was studied with four different buffers:
NH₄HCO₃/HAc, TRIS-HCl, Hepes, and PBS. The experiments were carried out with equimolar MBP-his6-AVI (produced by using pLink6) and copper ion concentration The final concentrations in the reaction mixture (35 µl) are shown in Table 6. Incubation time was ½h in RT.

**Table 6. Final concentrations in the reaction mixture (35 µl).**

| | 1 | 2 | 3 | 4 | 5 | 6 |
|---|---|---|---|---|---|---|
| LMW standard | x | | | | | |
| 3.2 µM MBP-his6-AVI | | x | x | x | x | x |
| 3.2 µM CuCl₂ | | | x | x | x | x |
| 3.4 mM Ascorbate | | | x | x | x | x |
| 1.1 mM H₂O₂ | | | x | x | x | x |
| 50 mM NH₄HCO₃/HAc | | x | x | | | |
| 50 mM TRIS-HCl | | | | x | | |
| 50 mM Hepes | | | | | x | |
| 50 mM PBS | | | | | | x |

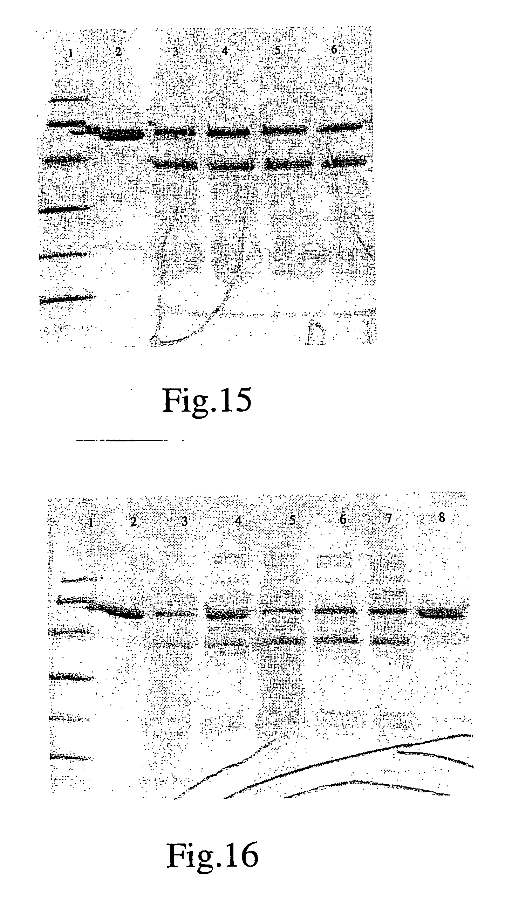

Results presented in figure 15. The cleavage was equally efficient regardless of which buffer was used.

### Example 8

### Effect of pH on cleavage

The effect of pH was studied in buffers: glycine-HCl (pH 3), NaAc/HAc (pH 5), Hepes (pH 7), Tris-HCl (pH 8), and glycine-NaOH (pH 10). For comparison, NH₄HCO₃/HAc, pH 8.6 was also used. The experiments were carried out with equimolar MBP-his6-AVI (produced by using pLink6) and copper ion concentration. The final concentrations in the reaction mixture (35 µl) are shown in Table 7. Incubation time was ½ h in RT.

**Table 7. Final concentrations in the reaction mixture (35 µl).**

| | 1 | 2 | 3 | 4 | 5 | 6 | 7 | 8 |
|---|---|---|---|---|---|---|---|---|
| LMW standard | x | | | | | | | |
| 3.2 µM MBP-his6-AVI | | x | x | x | x | x | x | x |
| 3.2 µM CuCl₂ | | | x | x | x | x | x | x |
| 3.4 mM Ascorbate | | | x | x | x | x | x | x |
| 1.1 mM H₂O₂ | | | x | x | x | x | x | x |
| 17.1 mM NH₄HCO₃/HAc (pH 8.6) | | x | x | | | | | |
| 17.1 mM Glycine-HCl (pH 3) | | | | x | | | | |
| 17.1 mM NaAc/HAc (pH 5) | | | | | x | | | |
| 17.1 mM Hepes (pH 7) | | | | | | x | | |
| 17.1 mM TRIS-HCl (pH 8) | | | | | | | x | |
| 17.1 mM Glycine-NaOH (pH 10) | | | | | | | | x |

Results presented in figure 16. The pH has a minor role. Only at pH 10 is there a clear decrease in effectivity.

### Example 9

### Effect of linker composition.

The experiments were done MBP-His0-AVI (control, no histidines in the linker) (produced by using pLink7), MBP-His2-AVI (two histidines in the linker) (produced by using pLink10), MBP-His4-AVI (four histidines) (produced by using pLink8), and MBP-His6-AVI (six histidines) (produced by using pLink6) in 8.5 mM NH₄HCO₃/HAc buffer, pH 8.6. Copper ion (Cu²⁺) concentration was equimolar to the protein concentration. The final concentrations in the reaction mixture (35 µl) are shown in Table 8. Incubation time was 1h in RT.

**Table 8. Final concentrations in the reaction mixture (35 µl).**

| | 1 | 2 | 3 | 4 | 5 | 6 | 7 | 8 | 9 |
|---|---|---|---|---|---|---|---|---|---|
| LMW standard | x | | | | | | | | |
| 3.3 µM MBP-His0-AVI | | x | x | | | | | | |
| 3.3 µM MBP-His2-AVI | | | | x | x | | | | |
| 3.3 µM MBP-His4-AVI | | | | | | x | x | | |
| 3.3 µM MBP-His6-AVI | | | | | | | | x | x |
| 3.3 µM CuCl₂ | | | x | | x | | x | | x |
| 4.6 mM Ascorbate | | | x | | x | | x | | x |
| 1.1 mM H₂O₂ | | | x | | x | | x | | x |

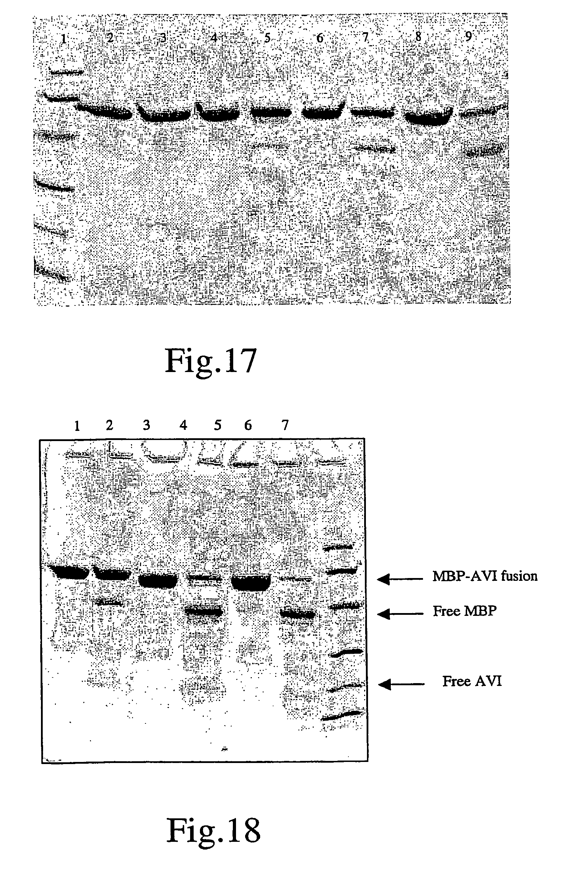

Results presented in figure 17. Six histidines in the linker is cleaved more efficiently than linker containing two or four histidines.

### Example 10

### Effect of linker composition

Comparison of increasing the linker to eight (MBP-His8-AVI) (produced by using pLink12) and inserting other amino acids inbetween His-residues (MBP-(HisXaa)6-AVI) (produced by using pLink13) was tested. Incubation as in example 7. All samples in ammonium acetate buffer at pH 7, 13 mM ascorbate, and 3.5 mM hydrogen peroxide .

| | | 1 | 2 | 3 | 4 | 5 | 6 | 7 |
|---|---|---|---|---|---|---|---|---|
| | LMW | | | | | | | X |
| 3.3 µM | MBP-His6-Avi | X | X | | | | | |
| 3.3 µM | MBP-His8-Avi | | | X | X | | | |
| 3.3 µM | MBP-(HisXaa)6-Avi | | | | | X | X | |
| 3.3 µM | CuCl2 | | X | | X | | X | |

Results presented in figure 18. A more efficient cleavage into free MBP and Avidin can be seen with the eight His and six (His-Xaa) repeats containing segments.

References:
1. Croft, L. R. Protein Sequence Analysis, 2nd Edition, John Wiley and Sons; B. Witkop, Advan. Prot. Chem. 16 (1961), 221
2. Kim, K., Rhee, S, and Stadtman, E.(1985) J. Biol Chem, 260. 15394-15397
3. Nord, K., Nilsson, J., Uhlen, M., Nygren, P.A. (1995) Protein Eng. 8, 601-608.
4. Oker-Blom C, Orellana A, Keinanen K. (1996) FEBS Lett., 389, 238-243.
5. Rana, T. and Meares, C. (1991). Proc Natl Acad Sci USA. 88, 10578-82.
6. Sambrook, J., Fritsch, E.F., and Maniatis, T. (1989) Molecular Cloning: A Laboratory Manual, 2nd Ed, Cold Spring Harbor Laboratory, Cold Spring Harbor, NY.

## Claims

1. A method for cleaving a protein or peptide at a specific site, **characterized in that** the method comprises the steps:
- constructing at a predetermined cleavage site of the protein or peptide an amino acid sequence of 2 to 20 amino acids, wherein the amino acid sequence comprises X₁ X₁ or repeats thereof or two or more repeats of X₁Yₙ , wherein n=1, X₁ is His and Yₙ is any amino acid, and
said amino acid sequence is cleavable in the presence of free metal ions,
said amino acid sequence does not exist naturally in the protein or peptide to be cleaved; and
- allowing said protein or peptide to react with the metal ion in a buffer, said buffer further comprising a reducing or oxidizing agent or agents.

2. The method according to claim 1, wherein the length of the amino acid sequence is 2 to 10 amino acids, preferably 4 to 8 amino acids.

3. The method according to claim 1 or 2, wherein the amino acid Yₙ is selected from the group comprising Cys, Lys and Trp.

4. The method according to any one of the preceding claims, wherein the amino acid Yₙ is His or Lys.

5. The method according to any one of the preceding claims, wherein the amino acid sequence comprises a sequence selected from the group
(His)₂, (His)₄ (SEQ ID NO:28), (His)₆ (SEQ ID NO:29), (His)₈ (SEQ ID NO:30) and His-Ser-His-Ala-His-Gly-His-Ala-His-Ser-His-Gly (SEQ ID NO:9).

6. The method according to any one of the preceding claims, wherein the metal ion is a ion of a metal selected from the group of transition metals, preferably from the group comprising Cu, Co, Ni, Fe, Mn, Cd, Pd, Rh, Ru, Pt, Cr and Zn.

7. The method according to any one of the preceding claims, wherein the metal ion is a ion of a metal selected from the group comprising Cu, Co, Mn, Cr, Ni , Fe and Zn, preferably from the group comprising Cu and Co.

8. The method according to any one of the preceding claims, wherein the metal ion is a ion of Cu.

9. The method according to any one of the preceding claims, wherein me protein to be cleaved is a recombinant protein.

10. The method according to any one of the preceding claims, wherein the amino acid sequence is constructed at a predetermined cleavage site by genetic engineering methods.

11. The method according to any one of the preceding claims, wherein the reaction is carried out in the presence of a reagent selected from the group comprising hydrogen peroxide, ascorbate and dithiothreitol or in the presence of a combination of these reagents.

## Patentansprüche

1. Verfahren zum Spalten eines Proteins oder Peptids an einer spezifischen Stelle, **dadurch gekennzeichnet, dass** das Verfahren die folgenden Schritte umfasst:
- Konstruieren einer Aminosäuresequenz mit 2 bis 20 Aminosäuren an einer vorher festgelegten Spaltstelle des Proteins oder Peptids, wobei die Aminosäuresequenz X₁X₁ oder Wiederholungen davon oder zwei oder mehrere Wiederholungen von X₁Yₙ umfasst, wobei n=1, X₁ His ist und Yₙ eine beliebige Aminosäure ist, und
die Aminosäuresequenz in der Gegenwart freier Metallionen spaltbar ist, die Aminosäuresequenz nicht natürlicherweise in dem zu spaltenden Protein oder Peptid existiert;
und
- Reagierenlassen des Proteins oder Peptids mit dem Metallion in einem Puffer, wobei der Puffer des Weiteren ein reduzierendes oder oxidierendes Agens oder Agenzien umfasst.

2. Verfahren nach Anspruch 1, wobei die Länge der Aminosäuresequenz 2 bis 10 Aminosäuren, vorzugsweise 4 bis 8 Aminosäuren ist.

3. Verfahren nach Anspruch 1 oder 2, wobei die Aminosäure Yₙ ausgewählt ist aus der Gruppe umfassend Cys, Lys und Trp.

4. Verfahren nach einem der vorangegangenen Ansprüche, wobei die Aminosäure Yₙ His oder Lys ist.

5. Verfahren nach einem der vorangegangenen Ansprüche, wobei die Aminosäuresequenz eine Sequenz umfasst, die ausgewählt ist aus der Gruppe (His)₂, (His)₄ (SEQ ID NO: 28), (His)₆ (SEQ ID NO: 29), (His)₈ (SEQ ID NO: 30) und His-Ser-His-Ala-His-Gly-His-Ala-His-Ser-His-Gly (SEQ ID NO: 9).

6. Verfahren nach einem der vorangegangenen Ansprüche, wobei das Metallion ein lon eines Metalls ausgewählt aus der Gruppe der Übergangsmetalle ist, vorzugsweise aus der Gruppe umfassend Cu, Co, Ni, Fe, Mn, Cd, Pd, Rh, Ru, Pt, Cr und Zn.

7. Verfahren nach einem der vorangegangenen Ansprüche, wobei das Metallion ein lon eines Metalls ist ausgewählt aus der Gruppe umfassend Cu, Co, Mn, Cr, Ni, Fe und Zn, vorzugsweise aus der Gruppe umfassend Cu und Co.

8. Verfahren nach einem der vorangegangenen Ansprüche, wobei das Metallion ein Cu-lon ist.

9. Verfahren nach einem der vorangegangenen Ansprüche, wobei das zu spaltende Protein ein rekombinantes Protein ist.

10. Verfahren nach einem der vorangegangenen Ansprüche, wobei die Aminosäuresequenz an einer vorher festgelegten Spaltstelle mittels Genmanipulationsverfahren konstruiert wird.

11. Verfahren nach einem der vorangegangenen Ansprüche, wobei die Reaktion ausgeführt wird in Gegenwart eines Reagens ausgewählt aus der Gruppe umfassend Wasserstoffperoxid, Ascorbat und Dithiothreit oder in Gegenwart einer Kombination dieser Reagenzien.

## Revendications

1. Procédé de clivage d'une protéine ou d'un peptide au niveau d'un site spécifique, **caractérisé en ce que** le procédé comprend les étapes suivantes :
- construire au niveau d'un site de clivage prédéterminé de la protéine ou du peptide une séquence d'acides aminés composée de 2 à 20 acides aminés, dans laquelle la séquence d'acides aminés comprend X₁X₁ ou des répétitions de celui-ci ou deux ou plusieurs répétitions de X₁Yₙ, où n = 1, X₁ est His et Yₙ est n'importe quel acide aminé, et
ladite séquence d'acides aminés peut être clivée en présence d'ions métalliques libres,
ladite séquence d'acides aminés n'existe pas naturellement dans la protéine ou le peptide à cliver ; et
- permettre à ladite protéine ou audit peptide de réagir avec l'ion métallique dans un tampon, ledit tampon comprenant en outre un ou des agent(s) réducteur(s) ou oxydant(s).

2. Procédé selon la revendication 1, dans lequel la longueur de la séquence d'acides aminés est de 2 à 10 acides aminés, de préférence de 4 à 8 acides aminés.

3. Procédé selon la revendication 1 ou 2, dans lequel l'acide aminé Yₙ est choisi dans le groupe comprenant Cys, Lys et Trp.

4. Procédé selon l'une quelconque des revendications précédentes, dans lequel l'acide aminé Yₙ est His ou Lys.

5. Procédé selon l'une quelconque des revendications précédentes, dans lequel la séquence d'acides aminés comprend une séquence choisie dans le groupe (His)₂, (His)₄(SEQ ID NO : 28), (His)₆(SEQ ID NO : 29), (His)₈(SEQ ID NO : 30) et (His)-Ser-His-Ala-His-Gly-His-Ala-His-Ser-His-Gly(SEQ ID NO : 9).

6. Procédé selon l'une quelconque des revendications précédentes, dans lequel l'ion métallique est un ion d'un métal choisi dans le groupe des métaux de transition, de préférence dans le groupe comprenant Cu, Co, Ni, Fe, Mn, Cd, Pd, Rh, Ru, Pt, Cr et Zn.

7. Procédé selon l'une quelconque des revendications précédentes, dans lequel l'ion métallique est un ion d'un métal choisi dans le groupe comprenant Cu, Co, Mn, Cr, Ni, Fe et Zn, de préférence dans le groupe comprenant Cu et Co.

8. Procédé selon l'une quelconque des revendications précédentes, dans lequel l'ion métallique est un ion de Cu.

9. Procédé selon l'une quelconque des revendications précédentes, dans lequel la protéine à cliver est une protéine recombinante.

10. Procédé selon l'une quelconque des revendications précédentes, dans lequel la séquence d'acides aminés est construite au niveau d'un site de clivage prédéterminé selon des procédés de génie génétique.

11. Procédé selon l'une quelconque des revendications précédentes, dans lequel la réaction est menée en présence d'un réactif choisi dans le groupe comprenant le peroxyde d'hydrogène, l'ascorbate et le dithiothréitol ou en présence d'une combinaison de ces réactifs.


### SEQUENCE LISTING

<110> Valtion teknillinen tutkimuskeskus
<120> A method for cleaving proteins
<130> VTT138PCT
<150> 2001050
   <151> 2003-07-09
<160> 30
<170> PatentIn version 3.1
<210> 1
   <211> 22
   <212> PRT
   <213> Artificial Sequence
<220>
   <223> amino acid sequence
   <400> 1
   Gly Ser Pro Thr Gly Ala Ser Thr His His His His His His Gly Ser
   1               5                   10                  15
   Pro Thr Gly Ala Ser Thr
               20
<210> 2
   <211> 22
   <212> PRT
   <213> Artificial sequence
<220>
   <223> amino acid sequence
   <400> 2
   Gly Ser Pro Thr Gly Ala Ser Thr Gly Gly Gly Gly Gly Gly Gly Ser
   1               5                   10                  15
   Pro Thr Gly Ala Ser Thr
               20
<210> 3
   <211> 22
   <212> PRT
   <213> Artificial Sequence
<220>
   <223> amino acid sequence
   <400> 3
   Gly Ser Pro Thr Gly Ala Ser Thr His His His His His His Gly Ser
   1               5                   10                  15
   Pro Thr Gly Ala Ser Thr
               20
<210> 4
   <211> 22
   <212> PRT
   <213> Artificial Sequence
<220>
   <223> amino acid sequence
   <400> 4
   Gly Ser Pro Thr Gly Ala Ser Thr Gly Ser Thr Gly Pro Ser Gly Ser
   1 5 10 15
   Pro Thr Gly Ala Ser Thr
   20
<210> 5
   <211> 20
   <212> PRT
   <213> Artificial sequence
<220>
   <223> amino acid sequence
   <400> 5
   Gly Ser Pro Thr Gly Ala Ser Thr His His His His Gly Ser Pro Thr
   1 5 10 15
   Gly Ala Ser Thr
   20
<210> 6
   <211> 18
   <212> PRT
   <213> Artificial sequence
<220>
   <223> amino acid sequence
   <400> 6
   Gly Ser Pro Thr Gly Ala Ser Thr His His Gly Ser Pro Thr Gly Ala
   1 5 10 15
   Ser Thr
<210> 7
   <211> 24
   <212> PRT
   <213> Artificial Sequence
<220>
   <223> amino acid sequence
   <400> 7
   Gly Ser Pro Thr Gly Ala Ser Thr His His His His His His His His
   1 5 10 15
   Gly Ser Pro Thr Gly Ala Ser Thr
   20
<210> 8
   <211> 27
   <212> PRT
   <213> Artificial Sequence
<220>
   <223> amino acid sequence
   <400> 8
   Gly Ser Pro Thr Gly Ala Ser Thr His Ser His Ala His Gly His Ala
   1 5 10 15
   His Ser His Gly Ser Pro Thr Gly Ala Ser Thr
   20 25
<210> 9
   <211> 12
   <212> PRT
   <213> Artificial sequence
<220>
   <223> amino acid sequence
   <400> 9
   His Ser His Ala His Gly His Ala His Ser His Gly
   1 5 10
<210> 10
   <211> 40
   <212> DNA
   <213> Artificial sequence
<220>
   <223> oligonucleotide
   <400> 10
   gcattggatt cgaattctta gctgaagcta aagtcttagc 40
<210> 11
<211> 34
   <212> DNA
   <213> Artificial sequence
<220>
   <223> oligonucleotide
   <400> 11
   gcattaagct tctattcgct ttttgccgga gtag 34
<210> 12
   <211> 69
   <212> DNA
   <213> Artificial sequence
<220>
   <223> oligonucleotide
   <400> 12
   cgggtagccc aaccggcgcg agcacccatc accatcacca tcacggtagc ccaaccggcg 60
   cgagcaccg 69
<210> 13
   <211> 77
   <212> DNA
   <213> Artificial sequence
<220>
   <223> oligonucleotide
   <400> 13
   aattcggtgc tcgcgccggt tgggctaccg tgatggtgat ggtgatgggt gctcgcgccg 60
   gttgggctac ccgagct 77
<210> 14
   <211> 69
   <212> DNA
   <213> Artificial sequence
<220>
   <223> oligonucleotide
   <400> 14
   cgggtagccc aaccggcgcg agcaccggcg gtggtggtgg cggcggtagc ccaaccggcg 60
   cgagcaccg 69
<210> 15
   <211> 77
   <212> DNA
   <213> Artificial sequence
<220>
   <223> oligonucleotide
   <400> 15
   aattcggtgc tcgcgccggt tgggctaccg ccgccaccac cagggccggt gctcgcgccg 60
   gttgggctac ccgagct 77
<210> 16
   <211> 33
   <212> DNA
   <213> Artificial sequence
<220>
   <223> oligonucleotide
   <400> 16
   gcattgaatt cgacccctcc aaggactcga agg 33
<210> 17
   <211> 33
   <212> DNA
   <213> Artificial sequence
<220>
   <223> oligonucleotide
   <400> 17
   gcattaagct tctactgctg aacggcgtcg agc 33
<210> 18
   <211> 69
   <212> DNA
   <213> Artificial sequence
<220>
   <223> oligonucleotide
   <400> 18
   cgggtagccc aaccggcgcg agcaccggca gcaccggtcc aagcggtagc ccaaccggcg 60
   cgagcaccg 69
<210> 19
   <211> 77
   <212> DNA
   <213> Artificial sequence
<220>
   <223> oligonucleotide
   <400> 19
   aattcggtgc tcgcgccggt tgggctaccg cttggaccgg tgctgccggt gctcgcgccg 60
   gttgggctac ccgagct 77
<210> 20
   <211> 63
   <212> DNA
   <213> Artificial sequence
<220>
   <223> oligonucleotide
   <400> 20
   cgggtagccc aaccggcgcg agcacccatc accatcacgg tagcccaacc ggcgcgagca 60
   ccg 63
<210> 21
   <211> 67
   <212> DNA
   <213> Artificial sequence
<220>
   <223> oligonucleotide
   <400> 21
   aattcggtgc tcgcgccggt tgggctaccg tgatggtgat gggtgctcgc gccggttggg 60
   ctacccg 67
<210> 22
   <211> 56
   <212> DNA
   <213> Artificial sequence
<220>
   <223> oligonucleotide
   <400> 22
   cgggtagccc aaccggcgcg agcacccatc acggtagccc aaccggcgcg agcacc 56
<210> 23
   <211> 65
   <212> DNA
   <213> Artificial sequence
<220>
   <223> oligonucleotide
   <400> 23
   aattcggtgc tcgcgccggt tgggctaccg tgatgggtgc tcgcgccggt tgggctaccc 60
   gagct 65
<210> 24
   <211> 75
   <212> DNA
   <213> Artificial sequence
<220>
   <223> oligonucleotide
   <400> 24
   cgggtagccc aaccggcgcg agcacccacc atcaccatca ccatcaccat ggtagcccaa 60
   ccggcgcgag caccg 75
<210> 25
   <211> 83
   <212> DNA
   <213> Artificial sequence
<220>
   <223> oligonucleotide
   <400> 25
   aattcggtgc tcgcgccggt tgggctacca tggtgatggt gatggtgatg gtgggtgctc 60
   gcgccggttg ggctacccga gct 83
<210> 26
   <211> 84
   <212> DNA
   <213> Artificial sequence
<220>
   <223> oligonucleotide
   <400> 26
   cgggtagccc aaccggcgcg agcacccata gccacgcgca tggccacgcg catagccacg 60
   gtagcccaac cggcgcgagc accg 84
<210> 27
   <211> 92
   <212> DNA
   <213> Artificial sequence
<220>
   <223> oligonucleotide
   <400> 27
   aattcggtgc tcgcgccggt tgggctaccg tggctatgcg cgtggccatg cgcgtggcta 60
   tgggtgctcg cgccggttgg gctacccgag ct 92
<210> 28
   <211> 4
   <212> PRT
   <213> Artificial Sequence
<220>
   <223> amino acid sequence
   <400> 28
   His His His His
   1
<210> 29
   <211> 6
   <212> PRT
   <213> Artificial sequence
<220>
   <223> amino acid sequence
   <400> 29
   His His His His His His
   1 5
<210> 30
   <211> 8
   <212> PRT
   <213> Artificial sequence
<220>
   <223> amino acid sequence
   <400> 30
   His His His His His His His His
   1 5